# EUROPÄISCHE PATENTSCHRIFT

(11) **EP 2 242 452 B1**
(45) Veröffentlichungstag und Bekanntmachung des Hinweises auf die Patenterteilung: **25.06.2014**
(21) Anmeldenummer: 09713591.7
(22) Anmeldetag: 03.02.2009
(51) Int. Cl.: A61F 2/40

(54) **SCHULTERGELENKPROTHESE, INSBESONDERE INVERSE SCHULTERGELENKPROTHESE**
SHOULDER JOINT PROSTHESIS, ESPECIALLY REVERSE SHOULDER JOINT PROSTHESIS
PROTHÈSE DE L ARTICULATION GLÉNO-HUMÉRALE, EN PARTICULIER PROTHÈSE INVERSÉE DE L ARTICULATION GLÉNO-HUMÉRALE

(30) Priorität: 21.02.2008 DE 102008010478
(43) Veröffentlichungstag der Anmeldung: 27.10.2010
(73) Patentinhaber: Smith & Nephew Orthopaedics AG, 6343 Rotkreuz (CH)
(72) Erfinder: SIMMEN, Beat, R., CH-8002 Zürich (CH); RINER, Marc, A., CH-5628 Aristau (CH); KEHRLI, Ernst, CH-6343 Rotkreuz (CH)
(74) Vertreter: Popp, Eugen
(86) Internationale Anmeldenummer: PCT/EP2009/000701
(87) Internationale Veröffentlichungsnummer: WO 2009/103413

(56) Entgegenhaltungen:
- EP-A- 1 402 854
- EP-A- 1 472 999
- EP-A- 1 607 068
- WO-A-2007/039820
- DE-A1-102008 021 110
- FR-A- 2 876 899

## Beschreibung

Die vorliegende Erfindung betrifft eine Schultergelenkprothese, insbesondere inverse Schultergelenkprothese gemäß dem Oberbegriff des Anspruches 1.

Verschiedene Schultergelenkserkrankungen können den Einbau eines künstlichen Schultergelenkes erforderlich machen. Der Anlaß für solch einen Eingriff sind neben den sichtbaren Veränderungen des Gelenkes im Röntgenbild oder Kernspin die Beschwerden des Patienten, wobei in erster Linie der starke Ruhe- und Belastungsschmerz zu nennen ist, der die Einnahme von Medikamenten erforderlich macht.

Neben dem künstlichen Ersatz von Hüft- und Kniegelenk hat die Schultergelenkendoprothetik in den letzten Jahren große Fortschritte gemacht. Bei der schmerzhaften Zerstörung des Schultergelenkes durch Arthrose (Gelenkverschleiß), Rheumatiode Arthritis (rheumatische Gelenkentzündung), Oberarmkopf-Nekrose (Absterben des Oberarmkopfes), oder Oberarmkopfbruch, ist es heute mit gutem Erfolg möglich, die Gelenkflächen von Oberarmkopf und Schulterpfanne durch Prothesen aus Titan und Polyethylen zu ersetzen.

Bei einem nicht unbeachtlichen Anteil der Patienten mit unbehandelter Rotatorenmanschettenmassenruptur (mindestens zwei gerissene Sehnen der Rotatorenmanschette) entwickelt sich eine Arthrose des Schultergelenkes. Bei der Rotatorenmanschettenmassenruptur verlagert sich der Oberarmkopf nach vorne-oben und verliert somit seine "Zentrierung" in der Gelenkpfanne. Zusätzlich kommt es hierbei zu einer Verkürzung und Schwächung des M. deltoideus, der im Fall der Rotatorenmanschettenmassenruptur noch eine Seithebung des Armes ermöglicht. In diesem Fall kann das Gelenk durch ein sogenanntes inverses Prothesen-System (inverse Delta-III-Prothese nach Grammont) ersetzt werden. Hierbei wird eine "Halbkugel" bzw. Glenosphäre auf die ursprüngliche Pfanne aufgeschraubt und eine konkav geformte "Humeruspfanne" im Oberarmknochen fixiert. Der Oberarm wird dadurch wieder nach "unten" verlagert und die Funktion des M. deltoideus wird verbessert.

Hier setzen nun die Überlegungen der vorliegenden Erfindung an:
Der Oberarmkopf ist zur Achse des Oberarms versetzt. Dies hat zur Folge, daß bei einer inversen Schultergelenkprothese der proximale Teil zu weit nach anterior verlagert ist. Dies führt zum einen zu einem relativ großen Knochenverlust an der anterioren Seite des proximalen Humerus und zum anderen zu einer nicht anatomischen Stellung des methaphysialen Teils des Implantats.

Für die Primärversorgung, bei der Humeruskopf und Glenoid unvertauscht bleiben, wird das vorgenannte Problem dadurch gelöst, daß die Gelenkköpfe am proximalen Ende des Humerus exzentrisch angeordnet sind, um eine anatomisch korrekte Stellung des Gelenkkopfes zu erhalten.

Für inverse Prothesen, bei der eine Glenosphäre auf einer scapularen Basisplatte montiert ist, ist es aus der WO 2007/039820 A2 zwar schon bekannt, die dem Humerusschaft zugeordnete Humeruspfanne relativ zum Schaft in Richtung anterior-posterior versetzt zu halten.

Dementsprechend liegt der vorliegenden Erfindung die Aufgabe zugrunde, eine Schultergelenkprothese, insbesondere inverse Schultergelenkprothese, deren dem Humerusschaft zugeordnete Gelenkteil anatomiegerecht eingestellt werden kann und die zu einem reduzierten Substanz(Knochen-)verlust führt.

Diese Aufgabe wird erfindungsgemäß durch die kennzeichnenden Merkmale des Anspruches 1 gelöst.

Ein wesentlicher Aspekt der vorliegenden Erfindung ist also die Möglichkeit einer individuellen Verlagerung des dem Humerusschaft zugeordneten Gelenkteils einer Schultergelenkprothese zumindest in Richtung anterior-posterior. Damit kann dieser Teil an die Anatomie des Patienten besser angepaßt werden. Dies gilt insbesondere für inverse Schultergelenkprothesen mit einer am Humerusschaft angeschlossenen Humeruspfanne.

Vorteilhafte Ausführungsformen und konstruktive Details der vorliegenden Erfindung sind in den Unteransprüchen beschrieben. Dabei sei darauf hingewiesen, daß das erfindungsgemäße Konzept nicht nur für inverse Schultergelenkprothesen, sondern auch für herkömmliche Schultergelenkprothesen zur primären Versorgung geeignet ist.

Sehr häufig ist es auch erforderlich, daß der dem Humerusschaft zugeordnete Gelenkteil der Schultergelenkprothese nach oben oder nach unten verlagert werden sollte, um eine anatomiegerechte Positionierung des Gelenks zu ermöglichen. Dies ist zum Beispiel dadurch möglich, daß zwischen schaftseitigem Gelenkteil, z.B. Humeruspfanne, und Schaft zwei Exzentrizitäten angeordnet und überlagert sind. Damit ist der dem Schaft zugeordnete Gelenkteil, z.B. bei inverser Konstruktion die Humeruspfanne, sowohl in Richtung anterior-posterior als auch in Richtung etwa senkrecht dazu bzw. medial-lateral versetzbar.

Die Fixierung der relativ zueinander versetzbaren Teile erfolgt durch eine Befestigungs- und Sicherungsschraube herkömmlicher Art.

Der dem Humerusschaft zugeordnete Gelenkteil der Schultergelenkprothese, bei inverser Ausbildung derselben die Humeruspfanne, ist gegenüber dem Schaft oder einem zwischen Schaft und Gelenkteil bzw. Humeruspfanne angeordneten Adapter entweder stufenlos oder schrittweise versetzbar.

Um eine indexierte Verlagerung des dem Schaft zugeordneten Gelenkteils zu erleichtern, insbesondere intraoperativ, weist dieser oder der Schaft oder ein dazwischen geschalteter Adapter an seiner dem jeweils anderen Teil der Prothese zugekehrten Seite eine im implantierten Zustand sich in Richtung anterior-posterior erstreckende Zahnreihe auf, die mit wenigstens einem Zahn am jeweils anderen Teil der Prothese zur schrittweisen Versetzung des dem Schaft zugeordneten Gelenkteils in Richtung anterior-posterior zusammenwirkt. Die Schrittweite beträgt vorzugsweise etwa 0,2 mm bis 0,8 mm, insbesondere etwa 0,5 mm. Der eine Zahn am jeweils anderen Teil der Prothese ist vorzugsweise ebenfalls durch eine Zahnreihe ersetzt, die sich anterior-posterior erstreckt. Damit erhält man bei schrittweiser Versetzung des schaftseitigen Gelenkteils gegenüber dem Schaft einen noch sichereren Halt der jeweils eingestellten Relativlage.

Des weiteren kann es vorteilhaft sein, wenn der dem Humerusschaft zugeordnete Gelenkteil, bei einer inversen Schultergelenkprothese die Humeruspfanne, relativ zum Schaft auch in Richtung proximal-distal versetzbar oder versetzt gehalten ist.

Um alle denkbaren Freiheitsgrade zur Verfügung zu stellen, kann der dem Humerusschaft zugeordnete Gelenkteil, bei einer inversen Schultergelenkprothese die Humeruspfanne, relativ zu Schaft auch noch kippbar gehalten sein, zumindest in Richtung anterior-posterior, vorzugsweise universalgelenkig.

Es sei an dieser Stelle auch noch erwähnt, daß der humerusseitige Gelenkteil, bei einer inversen Schultergelenkprothese die Humeruspfanne, vorzugsweise zweiteilig ausgebildet ist, d.h. einen Gelenkfläche definierenden Lagerteil aus Polyethylen, Keramik, Edelstahl od. dgl. humanverträglichem Lagerwerkstoff, und einen Lageraufnahmeteil umfaßt, über den der Anschluß am Humerusschaft erfolgt. Vorzugsweise ist der Lagerteil, der eine konvexe oder bei einer inversen Schultergelenkprothese konkave Gelenkfläche definiert, mit dem Lageraufnahmeteil verrastbar. Die Verrastung erfolgt so, daß bei Bedarf auch eine Revision des Lagerteils möglich ist, ohne daß im übrigen das Implantat verändert werden muß.

Alternativ zu der vorbeschriebenen Konstruktion ist es zur Lösung der gestellten Aufgabe auch denkbar, einen Satz von Gelenkteilen, bei einer inversen Schultergelenkprothese einen Satz von Humeruspfannen zur Verfügung zu stellen, deren Gelenkfläche, insbesondere deren geometrische Mittenachse relativ zum Schaft bzw. dessen geometrischer Längsmittenachse in Richtung posterior-anterior und/oder bei Bedarf medial-lateral und/oder bei Bedarf auch proximal-distal unterschiedlich weit versetzt angeordnet bzw. ausgebildet ist. Vorzugsweise umfaßt der Satz von Gelenkteilen insgesamt fünf Gelenkteile, wobei eines der Gelenkteile eine Gelenkfläche definiert, deren geometrische Mittenachse in der Ebene der geometrischen Längsmittenachse des Schaftes liegt. Die geometrischen Mittenachsen der Gelenkflächen der anderen Gelenkteile sind gegenüber der geometrischen Längsmittenachse des Schaftes unterschiedlich weit nach posterior und anterior, etc. versetzt.

Der maximale Versatz posterior-anterior und/oder medial-lateral und/oder proximal-distal liegt vorzugsweise im Bereich zwischen 3,0 mm bis 10,0 mm, insbesondere etwa im Bereich zwischen 4;0 mm und 8,0 mm. Diesbezüglich können mehrere Sätze von Gelenkteilen, z.B. Humeruspfannen, zur Verfügung gestellt werden, die sich jeweils durch einen maximalen Versatz posterior-anterior, etc. unterscheiden.

Die Schrittweite zwischen den einzelnen Versatzstufen liegt im Bereich von etwa 0,5 mm bis 2,0 mm, insbesondere etwa 1,0 mm bis 1,5 mm.

Auch ist es denkbar, einen Satz von Gelenkteilen, bei einer inversen Schultergelenkprothese einen Satz von Humeruspfannen zur Verfügung zu stellen, deren Gelenkflächen einen unterschiedlich großen Neigungswinkel relativ zum Schaft bzw. zu dessen Längsmittenachse aufweisen. Auch bei dieser Ausführungsform sollen also möglichst alle Freiheitsgrade dem Operateur zur Verfügung gestellt werden.

Nachstehend werden Ausführungsformen des humerusseitigen Teils einer erfindungsgemäß ausgebildeten inversen Schultergelenkprothese anhand der beigefügten Zeichnung näher erläutert. Diese zeigt in:
- Fig. 1: den dem Humerus zugeordneten Teil einer inversen Schultergelenkprothese in Ansicht von lateral, wobei die Humeruspfanne sich in der einen posterioren oder anterioren Endstellung befindet;
- Fig. 2: eine der Fig. 1 entsprechende Ansicht, wobei sich die Humeruspfanne in der gegenüber Fig. 1 entgegengesetzten Endstellung befindet;
- Fig. 3 u. 4: der dem Humerus zugeordnete Teil der inversen Schultergelenkprothese entsprechend den Fig. 1 und 2, jedoch jeweils von medial;
- Fig. 5 - 8: einen Humerusschaft, dem ein Satz von jeweils fünf Humeruspfannen zugeordnet ist, die eine relativ zum Schaft unterschiedlich weit anterior und posterior versetzte Gelenkfläche definieren, und zwar in Zusammenstellungsansicht von medial (Fig. 5), in Explosionsansicht von medial (Fig. 6), in Zusammenstellungsansicht von lateral (Fig. 7) und in Explosionsansicht von lateral (Fig. 8).

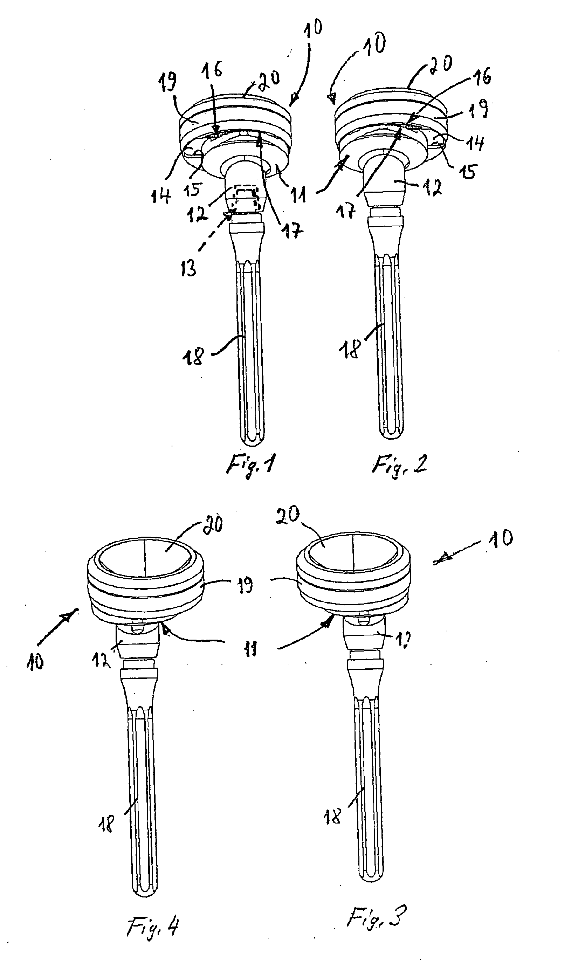

Der in den Figuren 1 bis 4 dargestellte, dem Humerus zugeordnete Teil einer inversen Schultergelenkprothese umfaßt eine Humeruspfanne 10, die einen vorzugsweise aus Polyethylen (PE) oder Keramik hergestellten Lagereinsatz 20 und aus humanverträglichem Material, z.B. Titan oder Titanlegierung hergestellten Lageraufnahmeteil 19 umfaßt. Diese Baueinheit 10 ist am proximalen Ende eines Humerusschaftes 19 angeschlossen, und zwar bei der dargestellten Ausführungsform unter Zwischenschaltung eines Schaft-Anschlußkörpers bzw. Adapters 11. Der Adapter 11 ist über eine an sich bekannte Konusverbindung 13 mit dem proximalen Ende des Schaftes 18 verbunden.

Die Humeruspfanne bzw. deren Lagereinsatz 20 wirkt im implantierten Zustand mit einer auf einer scapularen Basisplatte montierten Glenosphäre zusammen, und zwar unter Ausbildung einer vollständigen inversen Schultergelenkprothese.

Wie die Figuren 1 und 2, ebenso wie 3 und 4 sehr gut erkennen lassen, ist die Humeruspfanne 10 relativ zum Schaft 18, und zwar bei der konkreten Ausführungsform relativ zum Adapter 11 in vorbestimmter Richtung versetzbar gehalten, und zwar in Richtung anterior-posterior. Bei der dargestellten Ausführungsform erfolgt die Verlagerung der Humeruspfanne 10 schrittweise. Alternativ ist auch eine stufenlose Verlagerung möglich.

Die Humeruspfanne 10 bzw. deren Lageraufnahmeteil 19 einerseits und der Adapter 11 andererseits weisen an ihren einander zugekehrten Seiten komplementäre Längsführungsmittel auf. Konkret weist zu diesem Zweck die Humeruspfanne 10 bzw. deren Lageraufnahmeteil 19 an ihrer dem Adapter 11 zugekehrten Seite eine Vertiefung 14 mit einer sich in Richtung anterior-posterior erstreckenden Begrenzungswand 15 auf, die längsführend mit einem komplementären, hier nicht näher dargestellten Vorsprung an der der Humeruspfanne 10 zugewandten Seite des Adapters 11 zusammenwirkt. Auch eine entsprechend inverse Konstruktion ist selbstverständlich denkbar.

Des weiteren zeigen insbesondere die Figuren 1 und 2, daß sowohl die Humeruspfanne bzw. deren Lageraufnahmeteil 19 als auch der Adapter 11 an ihren einander zugekehrten Seiten jeweils eine sich in Verlagerungsrichtung, d.h. hier in Richtung anterior-posterior erstreckende Zahnreihe 16, 17 aufweist, die zur schrittweisen Versetzung der Humeruspfanne 10 relativ zum Schaft 18 bzw. zu dem zwischen Humeruspfanne 10 und Schaft 18 zwischengeschalteten Adapter 11 in durch die Längsführungsmittel vorbestimmter Richtung zusammenwirken.

Die dargestellte Konstruktion ist modular aufgebaut. Die Humeruspfanne 10 läßt sich intraoperativ einstellen.

Ergänzend sei zu der dargestellten Konstruktion noch erwähnt, daß an der dem Schaft 18 zugekehrten Seite des Adapters 11 ein Fortsatz 12 mit Innenkonus ausgebildet ist. Dieser Innenkonus wirkt mit einem am proximalen Ende des Schaftes 18 ausgebildeten Außenkonus zusammen, und zwar unter Ausbildung der bereits erwähnten Konusverbindung 13.

Die relative Lage zwischen Humeruspfanne 10 und Adapter 11 wird durch eine Fixierschraube, die hier nicht näher dargestellt ist, sichergestellt.

Durch die beschriebene Konstruktion ist es nicht mehr erforderlich, am proximalen Ende des Humerus anterior zusätzlich Knochenmaterial zu entfernen, um die Humeruspfanne anatomiegerecht zu plazieren. Des weiteren läßt sich durch die beschriebene Konstruktion das Gelenk patientenindividuell anatomiegerecht einstellen. Dies gilt insbesondere für eine Konstruktion, bei der die Humeruspfanne nicht nur in Richtung anterior-posterior, sondern auch in Richtung etwa senkrecht dazu versetzbar ist, also in Richtung medial-lateral.

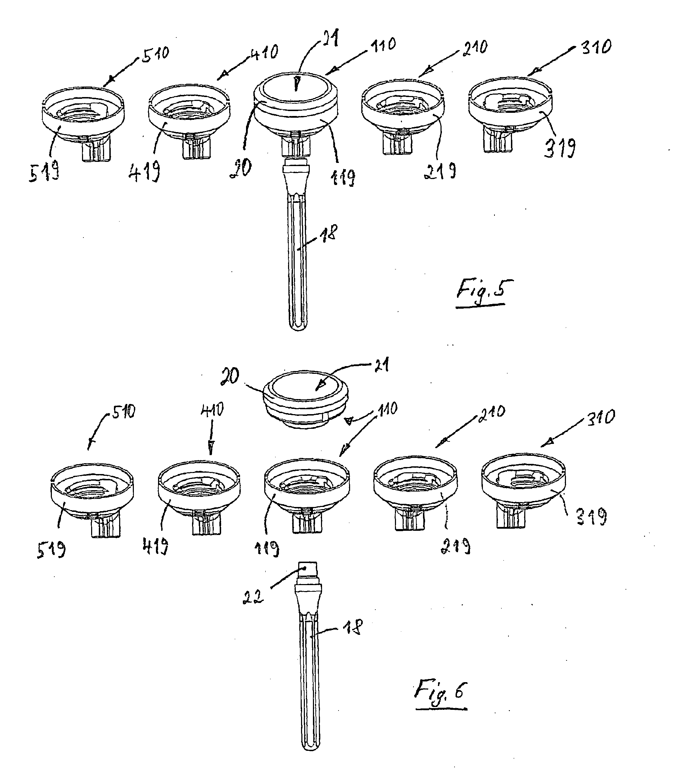

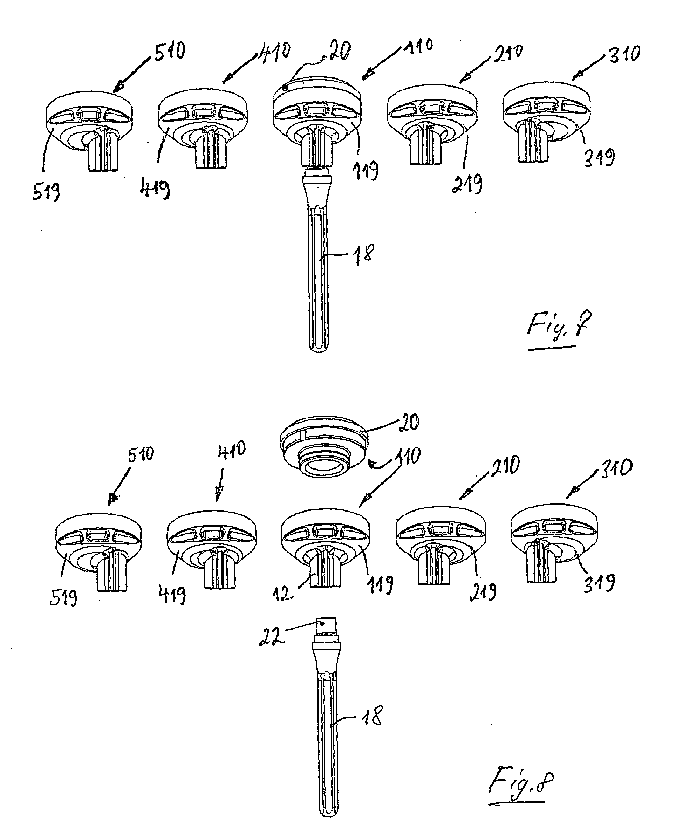

In den Figuren 5 bis 8 ist in unterschiedlichen Ansichten (von medial in den Figuren 5 und 6, und von lateral in den Figuren 7 und 8) und das eine Mal in Zusammenstellung (siehe Figuren 5 und 7) und das andere Mal in Explosion (siehe Figuren 6 und 8) ein Satz von Humeruspfannen 110, 210, 310, 410 und 510 dargestellt, deren Gelenkflächen 21, insbesondere deren geometrische Mittenachsen relativ zum Schaft 18 bzw. dessen geometrischer Längsmittenachse in Richtung posterior-anterior unterschiedlich weit versetzt angeordnet bzw. ausgebildet sind. Der größte Versatz wird durch die Humeruspfannen 310 und 510, ein weniger großer Versatz durch die Humeruspfannen 210 und 410 und kein Versatz durch die Humeruspfanne 110 definiert.

Der maximale Versatz posterior-anterior soll im Bereich zwischen 3,0 mm bis 10,0 mm, insbesondere etwa im Bereich zwischen 4,0 mm und 8,0 mm liegen. Es ist auch denkbar, mehrere Sätze von Humeruspfannen zur Verfügung zu stellen, die sich durch einen unterschiedlich maximalen Versatz in Richtung posterior-anterior und/oder medial-lateral und/oder proximal-distal auszeichnen. Dies hängt auch von der Größe des jeweiligen Patienten ab (Erwachsener oder Kind).

Die Schrittweite zwischen den einzelnen Versatzstufen liegt im Bereich von etwa 0,5 mm bis 2,0 mm, insbesondere im Bereich von etwa 1,5 mm. Auch dies hängt letztlich von den durch den Patienten vorgegebenen Umständen ab.

Im übrigen ist es auch denkbar, einen Satz von Gelenkteilen, bei einer inversen Schultergelenkprothese einen Satz von Humeruspfannen zur Verfügung zu stellen, deren Gelenkflächen 21 einen unterschiedlich großen Neigungswinkel relativ zum Schaft 18 bzw. zu dessen Längsmittenachse aufweisen. Diese Ausführungsform kann auch kombiniert werden mit einer Ausführungsform der hier dargestellten Form.

Auch hier sind die Humeruspfannen 110 bis 510 jeweils zweiteilig ausgebildet. Sie umfassen einen Lagereinsatz, bzw. ein Inlay 20 aus Polyethylen, Keramik, Edelstahl od. dgl. humanverträglichem Lagerwerkstoff einerseits und ein Lageraufnahmeteil 119, 219, 319, 419, 519 andererseits. Das Inlay 20 ist vorzugsweise im Lageraufnahmeteil 119 ff einrastbar, so daß es bei Bedarf auch durch ein anderes Lagerteil, insbesondere ein neues Lagerteil im Rahmen einer Revision ausgetauscht werden kann.

Die Verbindung zwischen Gelenkteil bzw. Humeruspfanne und Schaft 18 erfolgt über eine Konusverbindung entsprechend derjenigen, wie sie in Fig. 1 schematisch dargestellt ist. Zu diesem Zweck weist der Schaft 18 an seinem proximalen Ende einen Konus 22 auf, der innerhalb eines hier nicht näher dargestellten Innenkonus an einem unteren Fortsatz 12 der Lageraufnahmeteile 119 bis 519 in an sich bekannter Weise plazierbar ist.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen:

- 10: Humeruspfanne
- 110: Humeruspfanne
- 210: Humeruspfanne
- 310: Humeruspfanne
- 410: Humeruspfanne
- 510: Humeruspfanne
- 11: Adapter bzw. Schaft-Anschlußkörper
- 12: zentraler Fortsatz mit Innenkonus
- 13: Konusverbindung zwischen Schaft und Adapter
- 14: Vertiefung
- 15: Begrenzungswand der Vertiefung
- 16: Zahnreihe
- 17: Zahnreihe
- 18: Humerusschaft
- 19: Lageraufnahmeteil
- 119: Lagezaufnahmeteil
- 219: Lageraufnahmeteil
- 319: Lageraufnahmeteil
- 419: Lageraufnahmeteil
- 519: Lageraufnahmeteil
- 20: Lagereinsatz, vorzugsweise aus PE oder Keramik od. dgl. Gelenkflächenmaterial
- 21: Gelenkfläche
- 22: Konus

## Patentansprüche

1. Schultergelenkprothese, insbesondere inverse Schultergelenkprothese, mit einem am proximalen Ende eines Humerusschaftes (18) anschließbaren Gelenkteil, insbesondere einer Humeruspfanne (10), wobei der dem Humerusschaft (18) zugeordnete Gelenkteil, bei einer inversen Schultergelenkprothese die Humeruspfanne (10), relativ zum Schaft (18) zumindest in Richtung anterior-posterior versetzbar oder versetzt gehalten ist,
**dadurch gekennzeichnet, daß**
der dem Humerusschaft (18) zugeordnete Gelenkteil, bei einer inversen Konstruktion die Humeruspfanne (10), einerseits und der Schaft (18) oder ein zwischen diesem und dem Gelenkteil, z.B. Humeruspfanne (10) angeordneter Adapter (11) andererseits an ihren einander zugekehrten Seiten komplementäre Längsführungsmittel aufweisen.

2. Schultergelenkprothese nach Anspruch 1,
**dadurch gekennzeichnet, daß**
der dem Humerusschaft (18) zugeordnete Gelenkteil, z.B. Humeruspfanne (10), stufenlos oder schrittweise versetzbar ist.

3. Schultergelenkprothese nach Anspruch 1 oder 2,
**dadurch gekennzeichnet, daß**
der humerusseitige Teil der Schultergelenkprothese modular aufgebaut ist, insbesondere bestehend aus
- einem Gelenkteil, z.B. Humeruspfanne (10),
- einem Adapter (11), und
- einem Schaft (18).

4. Schultergelenkprothese nach Anspruch 1,
**dadurch gekennzeichnet, daß**
die Längsführungsmittel eine am Gelenkteil, z.B. an der Humeruspfanne (10) ausgebildete Vertiefung (14) mit wenigstens einer sich in Richtung anterior-posterior erstreckenden Begrenzungswand (15) und einen am zugeordneten Gegenstück, z.B. Adapter (11) ausgebildeten komplementären Vorsprung (15), oder umgekehrt umfassen.

5. Schultergelenkprothese nach einem der Ansprüche 1 bis 4,
**dadurch gekennzeichnet, daß**
der dem Schaft (18) zugeordnete Gelenkteil, z.B. Humeruspfanne (10), oder der Schaft (18) bzw. ein zwischen Schaft (18) und Gelenkteil, z.B. Humeruspfanne (10) angeordneter Adapter (11) an der dem jeweils anderen Teil der Prothese zugekehrten Seite eine sich in Richtung anterior-posterior erstreckende Zahnreihe (16 und/oder 17) aufweist, die mit wenigstens einem Zahn am jeweils anderen Teil der Prothese zur schrittweisen Versetzung des dem Humerus zugeordneten Gelenkteils, z.B. Humeruspfanne (10) in Richtung anterior-posterior zusammenwirkt.

6. Schultergelenkprothese nach einem der Ansprüche 1 bis 5,
**dadurch gekennzeichnet, daß**
der humerusseitige Gelenkteil, z.B. die Humeruspfanne (10), durch wenigstens eine Schraube am Schaft (18) oder einem zwischen diesem und dem Gelenkteil angeordneten Adapter (11) in vorbestimmter Relativlage fixierbar und gehalten ist.

7. Schultergelenkprothese nach einem der Ansprüche 1 bis 6,
**dadurch gekennzeichnet, daß**
der humerusseitige Gelenkteil, z.B. die Humeruspfanne (10), auch in Richtung etwa senkrecht zu anterior-posterior, d.h. etwa medial-lateral relativ zum Schaft (18) versetzbar oder versetzt gehalten ist.

8. Schultergelenkprothese nach einem der Ansprüche 1 bis 7,
**dadurch gekennzeichnet, daß**
zwischen humerusseitigem Gelenkteil, z.B. Humeruspfanne (10), und Schaft (18) und/oder einem dazwischengeschalteten Adapter (11) zwei Exzentrizitäten überlagert sind, so daß der Gelenkteil, z.B. die Humeruspfanne (10), sowohl in Richtung anterior-posterior als auch in Richtung etwa senkrecht dazu bzw. medial-lateral verlagerbar ist.

9. Schultergelenkprothese nach einem der Ansprüche 1 bis 8,
**dadurch gekennzeichnet, daß**
der dem Humerusschaft (18) zugeordnete Gelenkteil, bei einer inversen Schultergelenkprothese die Humeruspfanne (10), relativ zum Schaft (18) in Richtung proximal-distal versetzbar oder versetzt gehalten ist.

10. Schultergelenkprothese, insbesondere nach Anspruch 1,
**dadurch gekennzeichnet, daß**
daß ein Satz von Gelenkteilen, bei einer inversen Schultergelenkprothese ein Satz von Humeruspfannen (110, 210, 310, 410, 510) zur Verfügung gestellt ist, deren Gelenkflächen (21), insbesondere deren geometrische Mittenachsen relativ zum Schaft (18) bzw. dessen geometrischer Längsmittenachse in Richtung posterior-anterior und/oder medial-lateral und/oder proximal-distal unterschiedlich weit versetzt angeordnet bzw. ausgebildet sind (s. Fig. 5-8).

11. Schultergelenkprothese nach Anspruch 10,
**dadurch gekennzeichnet, daß**
der maximale Versatz posterior-anterior und/oder medial-lateral und/oder proximal-distal im Bereich zwischen 3,0 mm bis 10,0 mm, insbesondere etwa im Bereich zwischen 4,0 mm und 8,0 mm liegt.

12. Schultergelenkprothese nach Anspruch 10 oder 11,
**dadurch gekennzeichet, daß**
die Schrittweite zwischen den einzelnen Versatzstufen im Bereich von etwa 0,5 mm bis 2,0 mm, insbesondere etwa 1,0 mm bis 1,5 mm liegt.

13. Schultergelenkprothese, insbesondere nach einem der Ansprüche 1 bis 12,
**dadurch gekennzeichnet, daß**
ein Satz von Gelenkteilen, bei einer inversen Schultergelenkprothese ein Satz von Humeruspfannen (110, 210, 310, 410, 510) zur Verfügung gestellt ist, deren Gelenkflächen (21) einen unterschiedlich großen Neigungswinkel relativ zum Schaft (18) bzw. zu dessen Längsmittenachse aufweisen.

14. Schultergelenkprothese, insbesondere nach einem der Ansprüche 1 bis 12,
**dadurch gekennzeichnet, daß**
der dem Humerusschaft (18) zugeordnete Gelenkteil, bei einer inversen Schultergelenkprothese die Humeruspfanne (10) relativ zum Schaft (18) zumindest in Richtung anterior-posterior, insbesondere universal gelenkig kippbar gehalten ist.

15. Schultergelenkprothese nach einem der Ansprüche 1 bis 14,
**dadurch gekennzeichnet, daß**
der humerusseitige Gelenkteil, bei einer inversen Schultergelenkprothese die Humeruspfanne (10; 110, 210, 310, 410, 510) ein separat einsetzbares, insbesondere verrastbares Inlay (20) aus Polyethylen, Keramik, Edelstahl od. dgl. humanverträglichem Lagerwerkstoff umfaßt, welches die Gelenkfläche (21) dieses Gelenkteils definiert.

## Claims

1. A shoulder-joint prosthesis, especially an inverse shoulder-joint prosthesis, with a joint part, especially a humerus socket (10), capable of being connected at the proximal end of a humerus shaft (18) wherein the joint part assigned to the humerus shaft (18), in the case of an inverse shoulder-joint prosthesis, the humerus socket (10), is held relative to the shaft (18) in a manner capable of being offset or in an offset manner at least in the anterior-posterior direction,
**characterised in that**,
on the one side, the joint part assigned to the humerus shaft (18), in the case of an inverse construction, the humerus socket (10), and, on the other side, the shaft (18) or an adapter (11) arranged between the latter and the joint part, for example, the humerus socket (10), provide complementary longitudinal guide means on their mutually facing sides.

2. The shoulder-joint prosthesis according to claim 1,
**characterised in that**
the joint part, for example, humerus socket (10) assigned to the humerus shaft (18) can be offset in a continuously adjustable or stepwise manner.

3. The shoulder-joint prosthesis according to claim 1 or 2,
**characterised in that**
the humerus-side part of the shoulder-joint prosthesis is constructed in a modular manner, especially comprising:
- a joint part, for example, humerus socket (10),
- an adapter (11), and
- a shaft (18).

4. The shoulder-joint prosthesis according to claim 1,
**characterised in that**
the longitudinal guide means comprise a recess (14) embodied in the joint part, for example, in the humerus socket (10), with at least one limiting wall (15) extending in the anterior-posterior direction and a complementary projection (15) embodied on the assigned counterpart, for example, adapter (11), or vice versa.

5. The shoulder-joint prosthesis according to any one of claims 1 to 4,
**characterised in that**
the joint part, for example, humerus socket (10) assigned to the shaft (18), or the shaft (18) or respectively an adapter (11) arranged between shaft (18) and joint part, for example, humerus socket (10), provides a row of teeth (16 and/or 17) extending in the anterior-posterior direction at the side facing towards the respectively other part of the prosthesis, which cooperates with at least one tooth in the respectively other part of the prosthesis for the stepwise offsetting of the joint part, for example, humerus socket (10), assigned to the humerus in the anterior-posterior direction.

6. The shoulder-joint prosthesis according to any one of claims 1 to 5,
**characterised in that**
the humerus-side joint part, for example, the humerus socket (10), can be fixed and is held in a predetermined relative position by at least one screw in the shaft (18) or an adapter (11) arranged between the latter and the joint part.

7. The shoulder-joint prosthesis according to any one of claims 1 to 6,
**characterised in that**
the humerus-side joint part, for example, the humerus socket (10), can also be offset or held offset in the direction approximately perpendicular to anterior-posterior, that is, approximately medial-lateral relative to the shaft (18).

8. The shoulder-joint prosthesis according to any one of claims 1 to 7,
**characterised in that**,
between the humerus-side joint part, for example, humerus socket (10), and shaft (18) and/or an adapter (11) connected between the latter, two eccentricities are superposed, so that the joint part, for example, the humerus socket (10), is displaceable both in the anterior-posterior direction and also in the direction approximately perpendicular to the latter or respectively in the medial-lateral direction.

9. The shoulder-joint prosthesis according to any one of claims 1 to 8,
**characterised in that**
the joint part assigned to the humerus shaft (18), in the case of an inverse shoulder-joint prosthesis, the humerus socket (10), can be offset or held offset relative to the shaft (18) in the proximal-distal direction.

10. The shoulder-joint prosthesis, especially according to claim 1,
**characterised in that**
a set of joint parts, in the case of an inverse shoulder-joint prosthesis, a set of humerus sockets (110, 210, 310, 410, 510) is provided, of which the joint surfaces (21), especially their geometric mid-axes, are arranged or respectively embodied offset by a different distance relative to the shaft (18) or respectively its geometric, longitudinal mid-axis in the posterior-anterior and/or the medial-lateral and/or the proximal-distal direction (see Figs. 5-8).

11. The shoulder-joint prosthesis according to claim 10,
**characterised in that**
the maximal posterior-anterior and/or medial-lateral and/or proximal-distal offset is disposed in the range between 3.0 mm to 10.0 mm, especially approximately in the range between 4.0 mm and 8.0 mm.

12. The shoulder-joint prosthesis according to claim 10 or 11,
**characterised in that**
the step width between the individual offset steps is disposed in the range from approximately 0.5 mm to 2.0 mm, especially approximately 1.0 mm to 1.5 mm.

13. The shoulder-joint prosthesis, especially according to any one of claims 1 to 12,
**characterised in that**
a set of joint parts, in the case of an inverse shoulder-joint prosthesis, a set of humerus sockets (110, 210, 310, 410, 510) is provided, of which the joint surfaces (21) provide a differently large angle of inclination relative to the shaft (18) or respectively to its longitudinal mid-axis.

14. The shoulder-joint prosthesis, especially according to any one of claims 1 to 12,
**characterised in that**
the joint part assigned to the humerus shaft (18), in the case of an inverse shoulder-joint prosthesis, the humerus socket (10), is held in a tiltable manner relative to the shaft (18), at least in the anterior-posterior direction, especially universally articulated.

15. The shoulder-joint prosthesis according to any one of claims 1 to 14,
**characterised in that**
the humerus-side joint part, in the case of an inverse shoulder-joint prosthesis, the humerus socket (10; 110, 210, 310, 410, 510,), comprises a separately insertable, especially lockable, inlay (20) made of polyethylene, ceramic, stainless steel or similar human-compatible bearing material, which defines the joint surface (21) of this joint part.

## Revendications

1. Prothèse d'articulation d'épaule, plus particulièrement prothèse d'articulation d'épaule inversée, avec une partie d'articulation pouvant être raccordée à l'extrémité proximale d'un pivot huméral (18), plus particulièrement d'une cavité humérale (10), la partie de l'articulation correspondant au pivot huméral (18), c'est à dire la cavité humérale (10) dans le cas d'une prothèse d'articulation d'épaule inversée, pouvant être décalée ou étant maintenue décalée par rapport au pivot (18) au moins dans la direction antérieure-postérieure,
**caractérisée en ce que** la partie d'articulation correspondant au pivot huméral (18), c'est à dire la cavité humérale (10) dans le cas d'une construction inversée, d'une part, et le pivot (18) ou un adaptateur (11) disposé entre celui-ci et la partie d'articulation, par exemple la cavité humérale (10), d'autre part, présentent, au niveau de leurs côtés orientés l'un vers l'autre des moyens de guidage longitudinaux complémentaires.

2. Prothèse d'articulation d'épaule selon la revendication 1, **caractérisée en ce que** la partie d'articulation correspondant au pivot huméral (18), par exemple la cavité humérale (10) peut être décalée de manière continue ou par pas successifs.

3. Prothèse d'articulation d'épaule selon la revendication 1 ou 2, **caractérisée en ce que** la partie de la prothèse d'articulation d'épaule située du côté de humérus est conçue de manière modulaire et comprend, plus particulièrement :
- une partie d'articulation, par exemple une cavité humérale (10),
- un adaptateur (11) et
- un pivot (18).

4. Prothèse d'articulation d'épaule selon la revendication 1, **caractérisée en ce que** les moyens de guidage longitudinaux comprennent un creux (14) réalisé sur la partie d'articulation, par exemple sur la cavité humérale (10), avec au moins une paroi de limitation (15) s'étendant dans la direction antérieure-postérieure une saillie (15) complémentaire prévue sur la pièce correspondante, par exemple l'adaptateur (11) ou inversement.

5. Prothèse d'articulation d'épaule selon l'une des revendications 1 à 4, **caractérisée en ce que** la partie d'articulation correspondant au pivot (18), par exemple la cavité humérale (10), ou le pivot (18) ou un adaptateur (11) disposé entre le pivot (18) et la partie d'articulation, par exemple la cavité humérale (10), comprend, sur le côté orienté vers l'autre partie de la prothèse, une rangée de dents (16 et/ou 17) s'étendant dans la direction antérieure-postérieure, qui interagit avec au moins une dent située sur l'autre partie de la prothèse pour le décalage par pas successifs de la partie d'articulation correspondant à l'humérus, par exemple la cavité humérale (10), dans la direction antérieure-postérieure.

6. Prothèse d'articulation d'épaule selon l'une des revendications 1 à 5, **caractérisée en ce que** la partie d'articulation située du côté de l'humérus, par exemple la cavité humérale (10) peut être fixée et est maintenue dans une position relative prédéterminée par au moins une vis sur le pivot (18) ou un adaptateur (11) disposé entre celui-ci et la partie d'articulation.

7. Prothèse d'articulation d'épaule selon l'une des revendications 1 à 6, **caractérisée en ce que** la partie d'articulation située du côté de l'humérus, par exemple la cavité humérale (10), peut également être décalée ou être maintenue décalée dans une direction approximativement perpendiculaire à la direction antérieure-postérieure, c'est-à-dire approximativement médiale-latérale par rapport au pivot (18).

8. Prothèse d'articulation d'épaule selon l'une des revendications 1 à 7, **caractérisée en ce que**, entre la partie d'articulation située du côté de l'humérus, par exemple la cavité humérale (10), et le pivot (18) et/ou un adaptateur (11) inséré entre les deux, sont superposées deux excentricités, de façon à ce que la partie d'articulation, par exemple la cavité humérale (10), soit mobile aussi bien dans la direction antérieure-postérieure que dans la direction approximativement perpendiculaire à celle-ci ou médiale-latérale.

9. Prothèse d'articulation d'épaule selon l'une des revendications 1 à 8, **caractérisée en ce que** la partie d'articulation correspondant au pivot huméral (18), c'est-à-dire la cavité humérale (10) dans le cas d'une prothèse d'articulation d'épaule inversée, peut être décalée ou est maintenue décalée par rapport au pivot (18) dans la direction proximale-distale.

10. Prothèse d'articulation d'épaule selon la revendication 1, **caractérisée en ce qu'**un ensemble de parties d'articulation, c'est-à-dire un ensemble de cavités humérales (110, 210, 310, 410, 510) dans le cas d'une prothèse d'articulation d'épaule inversée, est mis à disposition, dont les surfaces d'articulation (21), plus particulièrement leurs axes géométriques centraux sont disposés ou conçus avec un décalage différent par rapport au pivot (18) ou à son axe géométrique central longitudinal dans la direction antérieure-postérieure et/ou médiale-latérale et/ou proximale/distale (voir figure 5-8).

11. Prothèse d'articulation d'épaule selon la revendication 10, **caractérisée en ce que** le décalage antérieur-postérieur et/ou médial/latéral et/ou proximal-distal maximal est de l'ordre de 3,0 mm à 10,0 mm, plus particulièrement de l'ordre d'environ 4,0 mm à 8,0 mm.

12. Prothèse d'articulation d'épaule selon la revendication 10 ou 11, **caractérisée en ce que** le pas entre les différentes étapes de décalage est de l'ordre d'environ 0,5 mm à 2,0 mm, plus particulièrement de l'ordre d'environ 1,0 mm à 1,5 mm.

13. Prothèse d'articulation d'épaule selon l'une des revendications 1 à 12, **caractérisée en ce qu'**un ensemble de parties d'articulation, c'est-à-dire un ensemble de cavités humérales (110, 210, 310, 410, 510) dans le cas d'une prothèse d'articulation d'épaule inversée, est mis à disposition, dont les surfaces d'articulation (21) présentent chacune un angle d'inclinaison de grandeur différente par rapport au pivot (18) ou à son axe central longitudinal.

14. Prothèse d'articulation d'épaule selon l'une des revendications 1 à 12, **caractérisée en ce que** la partie d'articulation correspondant au pivot huméral (18), c'est-à-dire la cavité humérale (10) dans le cas d'une prothèse d'articulation d'épaule inversée, est maintenue de manière universellement basculante et articulée par rapport au pivot (18) au moins dans la direction antérieure-postérieure.

15. Prothèse d'articulation d'épaule selon l'une des revendications 1 à 14, **caractérisée en ce que** la partie d'articulation située du côté de l'humérus, c'est-à-dire la cavité humérale (10 ; 110, 210, 310, 410, 510) dans le cas d'une prothèse d'articulation d'épaule inversée, comprend un élément encastrable (20) insérable séparément, plus particulièrement encliquetable, en polyéthylène, céramique, acier inoxydable ou autre matériau compatible avec l'organisme humain, qui définit la surface d'articulation (21) de cette partie d'articulation.
